(19) Europäisches Patentamt
European Patent Office
Office européen des brevets

(11) **EP 3 603 798 A1**

(12) **EUROPEAN PATENT APPLICATION**
published in accordance with Art. 153(4) EPC

(43) Date of publication:
**05.02.2020 Bulletin 2020/06**

(51) Int Cl.:
**B01J 20/285** (2006.01)  **B01D 15/36** (2006.01)
**B01D 15/38** (2006.01)  **B01J 20/24** (2006.01)
**B01J 20/281** (2006.01)

(21) Application number: **18776146.5**

(22) Date of filing: **29.03.2018**

(86) International application number:
**PCT/JP2018/013304**

(87) International publication number:
**WO 2018/181738 (04.10.2018 Gazette 2018/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.03.2017  JP 2017067569**

(71) Applicant: **Hitachi Chemical Company, Ltd.
Chiyoda-ku
Tokyo 100-6606 (JP)**

(72) Inventors:
• **KAWAGUCHI Akiko
Tokyo 100-6606 (JP)**
• **WATANABE Masaru
Tokyo 100-6606 (JP)**
• **KAWAUCHI Fumihiko
Tokyo 100-6606 (JP)**
• **GOTOH Yasushi
Tokyo 100-6606 (JP)**
• **YASUE Ken
Tokyo 100-6606 (JP)**
• **MIYAZAWA Emi
Tokyo 100-6606 (JP)**

(74) Representative: **Hoffmann Eitle
Patent- und Rechtsanwälte PartmbB
Arabellastraße 30
81925 München (DE)**

(54) **SEPARATION MATERIAL**

(57)    Disclosed is a separation material comprising hydrophobic polymer particles and a coating layer covering at least a portion of a surface of the hydrophobic polymer particles, wherein the coating layer comprises a hydrophilic polymer having hydroxy groups, and the hydrophilic polymer has a group represented by -NH-R-L or an epoxy group, wherein R represents a hydrocarbon group and L represents a carboxy group or an amino group.

## Description

### Technical Field

The present disclosure relates to a separation material.

### Background Art

Conventionally, when separating and purifying biopolymers represented by proteins, ion exchangers having a porous synthetic polymer as matrix, ion exchangers having a crosslinked gel of hydrophilic natural polymer as matrix, or the like are typically used. In the case of using the ion exchanger having a porous synthetic polymer as matrix packed in a column in chromatography, the pressure resistance during liquid passing tends to be excellent due to small volume change with salt concentration. However, in the case of using the ion exchanger in separation of proteins or the like, non-specific adsorption such as irreversible adsorption occurs based on hydrophobic interactions, so that there exists a problem that asymmetrization of peaks occurs, or proteins adsorbed on the ion exchanger remain adsorbed and cannot be collected due to the hydrophobic interactions.

On the other hand, an ion exchanger having a crosslinked gel of hydrophilic natural polymer represented by polysaccharides such as dextran and agarose as matrix has an advantage that there hardly exists non-specific adsorption of proteins. However, the ion exchanger significantly swells in an aqueous solution, having disadvantages that the volume change with ionic strength of the solution and the volume change between a free acid form and a loaded form are large, and the mechanical strength is insufficient. In particular, in the case of using the crosslinked gel in chromatography, there exist disadvantages that the pressure loss is large during liquid passing and the gel is compacted by liquid passing.

To overcome the disadvantages of the crosslinked gel of hydrophilic natural polymer, composite material holding a gel such as natural polymer gel in pores of a porous polymer is known in the field of peptide synthesis (e.g., refer to Patent Literature 1). By using such a composite material, the load factors of reactive substances increase, so that synthesis at a high yield can be achieved. Further, since the gel is enclosed with a rigid synthetic polymer material, even use in a column bed form causes no volume change, so that there exists an advantage that the pressure of flow passing through the column is maintained without change.

An ion exchanger of a hybrid copolymer prepared by filling the pores of a macroreticular copolymer with a gel of crosslinked copolymer synthesized from monomers is known (e.g., refer to Patent Literature 2). Although a crosslinked copolymer gel with a low crosslinking degree has problems such as pressure loss and volume change, formation of a hybrid copolymer improves liquid passing properties, achieving reduced pressure loss, enhanced ion exchange capacity, and improved leaking behavior.

Further, a complex filling material prepared by filling pores of an organic synthetic polymer substrate with a crosslinked gel of a hydrophilic natural polymer having a macromesh structure is proposed (e.g., refer to Patent Literatures 3 and 4).

There exists an affinity carrier comprising an affinity ligand bonded to an inorganic carrier such as silica (e.g., Patent Literature 5 and 6).

### Citation List

### Patent Literature

Patent Literature 1: US 4965289 A
Patent Literature 2: US 3966489 A
Patent Literature 3: JP H01-254247A
Patent Literature 4: US 5114577 A
Patent Literature 5: JP 2009-31277A
Patent Literature 6: US 4308254 A
Patent Literature 7: JP S60-169427A

### Summary of Invention

### Technical Problem

Conventional separation materials have problems that non-specific adsorption of proteins is high, and that column properties such as liquid passing properties are poor when used as a column.

An object of the present invention is therefore to provide a separation material with non-specific adsorption of proteins being reduced and excellent in column properties such as liquid passing properties when used as a column.

**Solution to Problem**

The present invention provides a separation material, a column and a method described in the following [1] to [25]:

[1] A separation material comprising hydrophobic polymer particles and a coating layer covering at least a portion of a surface of the hydrophobic polymer particles, wherein the coating layer comprises a hydrophilic polymer having hydroxy groups, and the hydrophilic polymer has a group represented by -NH-R-L or an epoxy group, wherein R represents a hydrocarbon group and L represents a carboxy group or an amino group.

[2] The separation material according to [1], wherein the separation material has an elastic modulus at 5% compressive deformation in water of 70 MPa or more.

[3] The separation material according to [1] or [2], wherein when water is passed through a column packed with the separation material such that the column has a pressure of 0.3 MPa, a water passing rate is 500 cm/h or more.

[4] The separation material according to any one of [1] to [3], wherein the hydrophobic polymer particles are particles comprising a polymer having a structural unit derived from a styrene monomer.

[5] The separation material according to any one of [1] to [4], wherein the hydrophilic polymer having hydroxy groups is a polysaccharide or a modified product thereof.

[6] The separation material according to any one of [1] to [5], wherein the hydrophilic polymer having hydroxy groups is one or more selected from the group consisting of dextran, agarose, pullulan, a modified product thereof, and a mixture thereof.

[7] The separation material according to any one of [1] to [6], wherein the hydrophilic polymer having hydroxy groups is crosslinked.

[8] The separation material according to any one of [1] to [7], wherein the separation material has an average particle size of 10 to 500 $\mu$m.

[9] The separation material according to any one of [1] to [8], wherein the separation material has a mode diameter in pore size distribution of 0.05 to 0.6 $\mu$m.

[10] The separation material according to any one of [1] to [9], wherein the separation material has a pore volume of 30 to 70 vol%.

[11] The separation material according to any one of [1] to [10], wherein the hydrophobic polymer particles have a porous structure.

[12] The separation material according to any one of [1] to [11], wherein the separation material has a specific surface area of 20 $m^2$/g or more.

[13] The separation material according to any one of [1] to [12], wherein the hydrophobic polymer particles have a coefficient of variation of a particle size of 3 to 15%.

[14] The separation material according to any one of [1] to [13], wherein an amount of the coating layer is 30 to 500 mg per g of the hydrophobic polymer particles.

[15] The separation material according to any one of [1] to [14], wherein the coating layer further has a ligand bonded to the hydrophilic polymer through a spacer comprising a structure derived from the group represented by -NH-R-L or a structure derived from the epoxy group.

[16] The separation material according to [15], wherein the ligand is covalently bonded to the spacer.

[17] The separation material according to [15] or [16], wherein the spacer is in a straight chain form.

[18] The separation material according to any one of [15] to [17], wherein the hydrophilic polymer is a polysaccharide or a modified product thereof, and a length of the spacer represented by the number of atoms from an oxygen atom derived from the hydroxy group in the hydrophilic polymer to an atom bonded to the ligand is 4 atoms or more and less than 50 atoms.

[19] The separation material according to any one of [15] to [18], wherein the ligand is bondable specifically to a portion of an immunoglobulin.

[20] The separation material according to any one of [15] to [19], wherein the ligand is one or more selected from the group consisting of protein A, protein G, protein L and functional variants thereof.

[21] A column comprising the separation material according to any one of [1] to [20].

[22] A method for separating a target molecule comprising: (a) contacting a solution comprising a target molecule with the separation material according to any one of [1] to [20] to adsorb the target molecule on the separation material; and (b) eluting the target molecule from the separation material on which the target molecule is adsorbed.

[23] The method according to [22], wherein the target molecule is at least a portion or a modified product of an immunoglobulin.

[24] The method according to [23], wherein the immunoglobulin is one or more selected from the group consisting of a monoclonal antibody and a polyclonal antibody.

[25] The separation material according to any one of [22] to [24], wherein the target molecule is a fusion protein comprising at least a portion of an Fc region of an immunoglobulin, or a modified product thereof.

**Advantageous Effects of Invention**

According to the present invention, a separation material with the non-specific adsorption of proteins being reduced and excellent in column properties such as liquid passing properties when used as a column can be provided.

**Description of Embodiments**

Preferred embodiments of the present invention are described in detail as follows, though the present invention is not limited to the embodiments.

<Separation material>

Separating material of the present embodiment comprises hydrophobic polymer particles and a coating layer covering at least a portion of the surface of the hydrophobic polymer particles, the coating layer comprising a hydrophilic polymer having hydroxy groups, the hydrophilic polymer having a group represented by -NH-R-L or an epoxy group, wherein R represents a hydrocarbon group and L represents a carboxy group or an amino group. The separation material of the present embodiment allows the non-specific adsorption of proteins to be reduced and has excellent liquid passing properties when packed in a column. Further, the separation material of the present embodiment has excellent ion exchange capacity, durability and alkali resistance, and it is conceivable that the adsorption capacity (dynamic adsorption capacity) is sufficiently high in practical use when packed in a column. The term "surface of hydrophobic polymer particles" in the present specification refers not only to the outer surface of the hydrophobic polymer particles but also the surface of pores in the internal part of the hydrophobic polymer particles.

[Hydrophobic polymer particles]

The hydrophobic polymer particles according to the present embodiment are particles comprising a polymer having hydrophobicity. No particular limitation is imposed on the production method of the hydrophobic polymer particles, and examples of the method include polymerizing a monomer capable of forming a polymer having hydrophobicity. As long as the monomer is capable of forming a polymer having hydrophobicity, no particular limitation is imposed thereon, and examples thereof include a styrene monomer. In other words, the hydrophobic polymer particles may comprise, for example, a hydrophobic polymer having a structural unit derived from a styrene monomer.

The hydrophobic polymer particles according to the present embodiment may have a porous structure. In other words, the hydrophobic polymer particles according to the present embodiment may be, for example, hydrophobic porous polymer particles. The porous polymer particles are, for example, particles comprising a polymer obtained by polymerizing a monomer in the presence of a porosity forming agent, which can be synthesized by conventional suspension polymerization, emulsion polymerization or the like. Specific examples of the monomer include the following polyfunctional monomers and monofunctional monomers.

Examples of the polyfunctional monomers include a divinyl compound such as divinylbenzene, divinylbiphenyl, divinylnaphthalene, and divinylphenanthrene. These polyfunctional monomers may be used singly or in combinations of two or more thereof. Among the above, it is preferable that the monomer contains divinylbenzene from the viewpoints of durability, acid resistance and alkali resistance.

Examples of the monofunctional monomers include styrene and derivatives thereof such as styrene, o-methylstyrene, m-methylstyrene, p-methylstyrene, $\alpha$-methylstyrene, o-ethylstyrene, m-ethylstyrene, p-ethylstyrene, 2,4-dimethylstyrene, p-n-butylstyrene, p-t-butylstyrene, p-n-hexylstyrene, p-n-octylstyrene, p-n-nonylstyrene, p-n-decylstyrene, p-n-dodecylstyrene, p-methoxystyrene, p-phenylstyrene, p-chlorostyrene, and 3,4-dichlorostyrene. These monofunctional monomers may be used singly or in combinations of two or more thereof. Among the above, use of styrene is preferred from the viewpoint of excellence in acid resistance and alkali resistance. Further, a styrene derivative having a functional group such as a carboxy group, an amino group, a hydroxy group and an aldehyde group may be also used.

Examples of the porosity forming agents include an organic solvent, which facilitates phase separation during polymerization and promotes making the particles porous, such as aliphatic or aromatic hydrocarbons, esters, ketones, ethers and alcohols. Examples of the porosity forming agents include toluene, xylene, cyclohexane, octane, butyl acetate, dibutyl phthalate, methyl ethyl ketone, dibutyl ether, 1-hexanol, 2-octanol, decanol, lauryl alcohol and cyclohexanol. These porosity forming agents may be used singly or in combinations of two or more thereof.

The amount of the porosity forming agent used may be 0 to 200 mass% relative to the total mass of the monomer. Depending on the amount of the porosity forming agent, the porosity of hydrophobic polymer particles can be controlled. Further, depending on the type of the porosity forming agent, the size and shape of the pores of hydrophobic polymer particles can be controlled.

Water used as solvent can be a porosity forming agent. In the case of using water as porosity forming agent, an oil-soluble surfactant is dissolved in the monomer to absorb water, so that particles can be made porous.

Examples of the oil-soluble surfactant used in porosity formation include: a sorbitan monoester of branched C16 to C24 fatty acids, linear unsaturated C16 to C22 fatty acids or linear saturated C12 to C14 fatty acids such as sorbitan monolaurate, sorbitan monooleate, sorbitan monomyristate or a sorbitan monoester derived from coconut fatty acid; a diglycerol monoester of branched C16 to C24 fatty acids, linear unsaturated C16 to C22 fatty acids or linear saturated C12 to C14 fatty acids such as diglycerol monooleate (e.g., diglycerol monoester of C18:1 (18 carbon atoms, one double bond) fatty acid), diglycerol monomyristate, diglycerol monoisostearate or a diglycerol monoester of coconut fatty acid; a diglycerol mono-aliphatic ether of branched C16 to C 24 alcohols (e.g., Guerbet alcohol), linear unsaturated C16 to C22 alcohols or linear saturated C12 to C14 alcohols (e.g., coconut fatty alcohol); and mixtures thereof.

Among these, a sorbitan monolaurate (e.g., SPAN (registered trade mark) 20, and a sorbitan monolaurate having a purity of preferably more than about 40%, more preferably more than about 50%, still more preferably more than about 70%), a sorbitan monooleate (e.g., SPAN (registered trade mark) 80 and a sorbitan monooleate having a purity of preferably more than about 40%, more preferably more than about 50%, still more preferably more than about 70%), a diglycerol monooleate (e.g., a diglycerol monooleate having a purity of preferably more than about 40%, more preferably more than about 50%, still more preferably more than about 70%), a diglycerol monoisostearate (e.g., diglycerol monoisostearate having a purity of preferably more than about 40%, more preferably more than about 50%, still more preferably more than about 70%), a diglycerol monomyristate (sorbitan monomyristate having a purity of preferably more than about 40%, more preferably more than about 50%, still more preferably more than about 70%), a diglycerol cocoyl (e.g., a lauryl group and a myristoyl group) ether, or a mixture thereof is preferred.

It is preferable that these oil-soluble surfactants be used in an amount in the range of 5 to 80 mass% relative to the total mass of the monomer. With a content of the oil-soluble surfactant of 5 mass% or more, the stability of water droplets is sufficiently enhanced, so that a large single hole is hardly formed. With a content of the oil-soluble surfactant of 80 mass% or less, the hydrophobic polymer particles more easily retain the shape after polymerization.

Examples of the aqueous medium for use in the polymerization reaction include water and a mixed media of water and a water-soluble solvent (e.g., lower alcohol). The aqueous medium may contain a surfactant. As the surfactant, any one of anionic, cationic, nonionic and amphoteric surfactants may be used.

Examples of the anionic surfactant include a fatty acid oil such as sodium oleate and potassium castor oil, an alkyl sulfate ester salt such as sodium lauryl sulfate and ammonium lauryl sulfate, an alkylbenzene sulfonate such as sodium dodecyl benzene sulfonate, an alkyl naphthalene sulfonate, an alkane sulfonate, a dialkyl sulfosuccinate such as sodium dioctyl sulfosuccinate, an alkenyl succinate (dipotassium salt), an alkyl phosphate ester salt, a naphthalene sulfonate formalin condensate, and a polyoxyethylene alkyl phenyl ether sulfate ester salt, a polyoxyethylene alkyl ether sulfate salt such as sodium polyoxyethylene lauryl ether sulfate, and a polyoxyethylene alkyl sulfate ester salt.

Examples of the cationic surfactant include an alkylamine salt such as laurylamine acetate and stearylamine acetate, and a quaternary ammonium salt such as lauryl trimethyl ammonium chloride.

Examples of the nonionic surfactant include a nonionic hydrocarbon surfactant such as polyethylene glycol alkyl ethers, polyethylene glycol alkylaryl ethers, polyethylene glycol esters, polyethylene glycol sorbitan esters, and polyalkylene glycol alkylamines or amides, a nonionic polyether-modified silicon surfactant such as polyethylene oxide adducts and polypropylene oxide adducts of silicon, and a nonionic fluorine surfactant such as perfluoroalkyl glycols.

Examples of the amphoteric surfactant include a hydrocarbon surfactant such as lauryl dimethylamine oxide, a phosphoric acid ester-based surfactant, and a phosphorous acid ester-based surfactant.

The surfactants may be used alone or in combination of two or more thereof. Among the surfactants described above, anionic surfactants are preferred from the viewpoint of dispersion stability during monomer polymerization.

Examples of polymerization initiators to be optionally added include an organic peroxide such as benzoyl peroxide, lauroyl peroxide, orthochloro benzoyl peroxide, orthomethoxy benzoyl peroxide, 3,5,5-trimethyl hexanoyl peroxide, tert-butyl peroxy-2-ethyl hexanoate, and di-tert-butyl peroxide; and an azo compound such as 2,2'-azobisisobutyronitrile, 1,1'-azobiscyclohexanecarbonitrile, and 2,2'-azobis(2,4-dimethylvaleronitrile). The polymerization initiator may be used in an amount in the range of 0.1 to 7.0 parts by mass relative to 100 parts by mass of the monomer.

The polymerization temperature may be appropriately selected depending on the types of the monomer and the polymerization initiator. The polymerization temperature is preferably 25 to 110°C, more preferably 50 to 100°C.

In the synthesis of the hydrophobic polymer particles, a polymer dispersion stabilizer may be used in order to improve the dispersion stability of particles.

Examples of the polymer dispersion stabilizer include polyvinyl alcohol, polycarboxylic acid, celluloses (hydroxyethyl cellulose, carboxymethyl cellulose, methyl cellulose, etc.), and polyvinylpyrrolidone, and an inorganic water-soluble polymer compound such as sodium tripolyphosphate may be used in combination. Among these, polyvinyl alcohol or polyvinyl pyrrolidone is preferred. It is preferable that the amount of the polymer dispersion stabilizer added be from 1 to 10 parts by mass relative to 100 parts by mass of the monomer.

In order to prevent the monomer from being polymerized alone, water-soluble polymerization inhibitors such as nitrites, sulfites, hydroquinones, ascorbic acids, water-soluble vitamin B's, citric acid, and polyphenols may be used.

The average particle size of the hydrophobic polymer particles and the separation material is preferably 500 $\mu$m or less, more preferably 300 $\mu$m or less, still more preferably 150 $\mu$m or less, furthermore preferably 100 $\mu$m or less. The average particle size of the hydrophobic polymer particles and separation material is preferably 10 $\mu$m or more, more preferably 30 $\mu$m or more, still more preferably 50 $\mu$m or more, from the viewpoint of improving the liquid passing properties. The average particle size of the hydrophobic polymer particles and the separation material is preferably 10 to 500 $\mu$m, more preferably from 30 to 300 $\mu$m, still more preferably 50 to 150 $\mu$m.

The coefficient of variation (C. V.) of the particle size of the hydrophobic polymer particles and the separation material is preferably 3 to 15%, more preferably 5 to 15%, still more preferably 5 to 10%, from the viewpoint of improving the liquid passing properties. Examples of methods for reducing C. V. of the particle size include monodispersing by an emulsifying apparatus such as a micro process server (manufactured by Hitachi, Ltd.).

The average particle size of the hydrophobic polymer particles or the separation material and C. V. (coefficient of variation) of the particle size can be obtained by the following measurement method.

1) The particles are dispersed in water (containing a dispersant such as a surfactant) by using an ultrasonic dispersing apparatus, so that a dispersion containing 1 mass% of particles is prepared.
2) By using a particle size distribution analyzer (Sysmex Flow, manufactured by Sysmex Corp.), the average particle size and C. V. (coefficient of variation) of the particle size are measured based on an image of about 10000 pieces of particles in the dispersion.

The pore volume (porosity) of the hydrophobic polymer particles and the separation material is preferably 30 vol% or more and 70 vol% or less, more preferably 40 vol% or more and 70 vol% or less, based on the total volume (including pore volume) of the hydrophobic polymer particles or the separation material.

It is preferable that the hydrophobic polymer particles and the separation material have pores having a pore size of 0.1 $\mu$m or more and less than 0.5 $\mu$m, i.e., macropores. The average pore size or mode pore size (mode diameter in pore size distribution) of the hydrophobic polymer particles and separation material is preferably 0.05 $\mu$m or more and 0.6 $\mu$m or less, more preferably 0.1 $\mu$m or more and less than 0.5 $\mu$m, still more preferably 0.2 $\mu$m or more and less than 0.5 $\mu$m. With an average pore size or a mode pore size of 0.05 $\mu$m or more, substances tend to easily enter the pore, and with an average pore size or a mode pore size of less than 0.6 $\mu$m, a sufficient specific surface area is obtained. The pore volume and the pore size can be adjusted by the porosity forming agent described above.

The specific surface area of the hydrophobic polymer particles and the separation material is preferably 20 $m^2$/g or more, more preferably 30 $m^2$/g or more. From the viewpoint of higher practicality, the specific surface area is still more preferably 35 $m^2$/g or more, and furthermore preferably 40 $m^2$/g or more. With a specific surface area of 20 $m^2$/g or more, the amount of a substance adsorbed to be separated tends to increase.

[Coating layer]

The coating layer according to the present embodiment comprises a hydrophilic polymer having hydroxy groups, and the hydrophilic polymer has a group expressed by -NH-R-L or an epoxy group, wherein R represents a hydrocarbon group, L represents a carboxy group or an amino group. By coating hydrophobic polymer particles with the hydrophilic polymer having hydroxy groups, non-specific adsorption of proteins can be suppressed, and the amount of proteins adsorbed can be improved. Also, when the hydrophilic polymer having a hydroxy group is crosslinked, the increase in column pressure can be further suppressed. Further, by introducing a specific functional group in the coating layer, the ligand can be immobilized. The group expressed by -NH-R-L or the epoxy group may be bonded to the hydrophilic polymer, for example, through hydroxy groups in the hydrophilic polymer.

(Hydrophilic polymer)

It is preferable that the hydrophilic polymer having hydroxy groups have 2 or more hydroxy groups in one molecule. Examples of the hydrophilic polymer having hydroxy groups include polysaccharides or a modified product thereof and polyvinyl alcohol or a modified polymer thereof. Examples of the polysaccharides include agarose, dextran, cellulose, pullulan, and chitosan. As the hydrophilic polymer having hydroxy groups, ones having an average molecular weight of 10000 or more such as ones having an average molecular weight of about 10000 to 200000 may be used. The hydrophilic polymer having hydroxy groups may be one or more selected from the group consisting of dextran, agarose, pullulan, modified products thereof and mixtures thereof.

It is preferable that the hydrophilic polymer having hydroxy groups be a modified product with a hydrophobic group introduced, from the viewpoint of improving the interfacial adsorption ability. Examples of the hydrophobic group include an alkyl group having 1 to 6 carbon atoms and an aryl group having 6 to 10 carbon atoms. Examples of the alkyl group having 1 to 6 carbon atoms include a methyl group, an ethyl group and a propyl group. Examples of the aryl group having 6 to 10 carbon atoms include a phenyl group and a naphthyl group. The hydrophobic group can be introduced by reacting a compound having a functional group reactive with hydroxy groups (e.g., epoxy group) and a hydrophobic group (e.g., glycidyl phenyl ether) with a hydrophilic polymer having hydroxy groups by a conventionally known method.

The content of the hydrophobic group in the modified product of the hydrophilic polymer with a hydrophobic group introduced is preferably 5 to 30 mass%, more preferably 10 to 20 mass%, still more preferably 12 to 17 mass%, from the viewpoints of balance between the retention of hydrophobic interaction force for adsorption to the particle surface and the suppression of non-specific adsorption of proteins.

(Method for forming coating layer)

The coating layer according to the present embodiment can be formed, for example, by the following method.

First, a solution of the hydrophilic polymer having hydroxy groups is adsorbed on the hydrophobic polymer particle surface. Although the solvent of the solution of the hydrophilic polymer having a hydroxy group is not particularly limited as long as it can dissolve the hydrophilic polymer having hydroxy groups, water is the most common. It is preferable that the concentration of the polymer dissolved in the solvent be 5 to 20 (mg/mL).

The hydrophobic polymer particles are impregnated with the solution. The impregnation method includes adding the hydrophobic polymer particles to the solution of a hydrophilic polymer having hydroxy groups to be left standing for a predetermined time. Although the impregnation time varies depending on the surface state of the hydrophobic polymer particles, an equilibrium state of the polymer concentration is obtained between inside and outside of the hydrophobic polymer particles usually after impregnation for 24 hours. An unadsorbed fraction of the hydrophilic polymer having hydroxy groups is then removed through washing with a solvent such as water or alcohol.

(Crosslinking treatment)

Subsequently, through addition of a crosslinking agent, a crosslinking reaction of the hydrophilic polymer having hydroxy groups adsorbed on the hydrophobic polymer particle surface is caused to form a crosslinked product. On this occasion, the crosslinked product has a three-dimensional crosslinked network having hydroxy groups.

Examples of the crosslinking agent include compounds having two or more functional groups that are active on hydroxy groups, i.e., epihalohydrins such as epichlorohydrin, dialdehyde compounds such as glutaraldehyde, diisocyanate compounds such as methylene diisocyanate, and glycidyl compounds such as ethylene glycol diglycidyl ether. Also, in the case of using a compound having an amino group such as chitosan as the hydrophilic polymer having hydroxy groups, a dihalide compound such as dichlorooctane may be used as the crosslinking agent.

In the crosslinking reaction, a catalyst is usually used. As the catalyst, a conventionally known one may be appropriately used in accordance with the type of the crosslinking agent, and, for example, in the case of using epichlorohydrin or the like as the crosslinking agent, an alkali such as sodium hydroxide is effective, and in the case of dialdehyde compounds, a mineral acid such as hydrochloric acid is effective.

The crosslinking reaction with a crosslinking agent is usually performed by addition of the crosslinking agent to a system including a separation material dispersed and suspended in a suitable medium. In the case of using polysaccharides as the hydrophilic polymer having hydroxy groups, the amount of the crosslinking agent added may be selected in the range of 0.1 to 100 moles relative to one monosaccharide unit as one mole, corresponding to the performance of the separation material. In general, as the amount of the crosslinking agent added is reduced, the coating layer tends to be easily peeled off from the hydrophobic polymer particles. In contrast, with an excessive amount of the crosslinking agent added, and besides with a high reaction rate with the hydrophilic polymer having hydroxy groups, properties of the raw material hydrophilic polymer having hydroxy groups tend to be impaired.

Although the amount of the catalyst used varies depending on the type of the crosslinking agent, in the normal case of using polysaccharides as the hydrophilic polymer having hydroxy groups, the amount used is in the range of 0.01 to 10 moles, preferably 0.1 to 5 moles, relative to one monosaccharide unit as one mole to form the polysaccharides.

For example, in the case of crosslinking conditions governed by temperature conditions, the temperature of the reaction system is raised to a reaction temperature, at which the crosslinking reaction occurs.

The medium for dispersing and emulsifying the hydrophobic polymer particles impregnated with the solution of the hydrophilic polymer having hydroxy groups or the like needs not to extract the polymer, the crosslinking agent or the like from the impregnating polymer solution, and besides, needs to be inactive on the crosslinking reaction. Specific examples of the media include water and, alcohol.

The crosslinking reaction is performed usually at a temperature in the range of 5 to 90°C, for 1 to 10 hours. Preferably, the temperature is in the range of 30 to 90°C.

When adjusting the degree of progress in crosslinking, the crosslinking reaction may be performed in stages. For example, the degree of progress in crosslinking may be adjusted by a re-crosslinking reaction of particles once crosslinked. The degree of progress in crosslinking is evaluated by the temperature at which 5% weight loss occurs in thermal decomposition. In the case of a high degree of progress in crosslinking, the weight reduction initiation temperature becomes high, while in the case of low degree of progress in crosslinking, weight loss initiation temperature becomes low.

The temperature at which 5% weight loss occurs is preferably 200 to 350°C, more preferably 220 to 330°C, still more preferably 230 to 320°C, from the viewpoint of maintaining the properties of the hydrophilic polymer having hydroxy groups. With an excessively low degree of crosslinking, the hydrophilic polymer having hydroxy groups tends to fall off from the particles, while with an excessively high degree of crosslinking, dynamic adsorption capacity decreases due to reduction in swellability of the polymer or the amount of functional groups, though the hydrophilic polymer having hydroxy groups is prevented from falling off from the particles.

After completion of the crosslinking reaction, the resulting particles were filtered and then washed with a hydrophilic organic solvent such as methanol and ethanol to remove unreacted polymers, medium for suspension and the like, so that particles of which at least a part of the surface of the hydrophilic polymer particles is covered with a coating layer comprising the hydrophilic polymer having hydroxy groups (hereinafter also referred to as "coated particles") are obtained.

(Introduction of functional group)

Into the coated particles described above, a functional group such as a carboxy group, an amino group, and an epoxy group may be introduced through hydroxy groups. The carboxy group and the amino group may be introduced as a group expressed by -NH-R-L. R represents a hydrocarbon group and L represents a carboxy group or an amino group. Examples of the hydrocarbon groups include alkylene groups having 1 to 10 carbon atoms. Into the coating layer of the separation material according to the present embodiment, a group expressed by -NH-R-L or an epoxy group may be introduced through hydroxy groups of the hydrophilic polymer. Since the coated particles have a functional group such as a carboxy group, an amino group, and an epoxy group, a ligand is able to be introduced into coated particles.

Examples of the method for introducing a functional group through hydroxy groups include a method of using a halogenated alkyl compound. Examples of the halogenated alkyl compounds include a monohalogenocarboxylic acid such as a monohalogenoacetic acid and a monohalogenopropionic acid, and a sodium salt thereof, and a primary, secondary or tertiary amine having at least one halogenated alkyl group such as diethylaminoethyl chloride. It is preferable that the halogenated alkyl compound be a sulfur compound, a bromide or a chloride.

Examples of the method for introducing an epoxy group as functional group include a method for reacting the coated particles with an epihalohydrin, ethylene glycol diglycidyl ether or 1,2,3,4-diepoxybutane. Examples of the epihalohydrin include epichlorohydrin, epibromohydrin, epiiodohydrin, $\beta$-methyl epichlorohydrin, $\alpha$-methyl epichlorohydrin and $\gamma$-methyl epichlorohydrin. It is preferable that the amount of the halogenated alkyl compound used be 0.2 mass% or more relative to the total mass of the coated particles. It is preferable that the reaction conditions be at 40 to 90°C for 0.5 to 12 hours.

Examples of the method for introducing a carboxy group as functional group include a method for reacting the coated particles with the epoxy group introduced with a carboxylic acid compound having an amino group. Examples of the carboxylic acid compound having an amino group include 3-aminopropanoic acid, 4-aminobutanoic acid, 5-aminopentanoic acid, 6-aminohexanoic acid and 7-aminoheptanoic acid. It is preferable that the amount of the carboxylic acid compound having an amino group used be 0.2 mass% or more relative to the total mass of the coated particles with an epoxy group introduced therein.

Examples of the method for introducing an amino group as functional group include a method for reacting the coated particles with the epoxy group introduced with a diamine compound. Examples of the diamine compounds include ethylenediamine, 1,3-diaminopropane, 1,4-diaminobutane, 1,6-diaminohexane, 1,7-diaminoheptane, and 1,8-diaminooctane. It is preferable that the amount of the diamine compound used be 0.2 mass% or more relative to the total mass of the coated particles with the epoxy group introduced.

The hydrophilic polymer in the coating layer in the present embodiment may have a structure derived from a group represented by -NH-R-L and/or a structure derived from an epoxy group as a spacer for bonding to a ligand. In other words, in the separation material according to the present embodiment, a ligand may be bonded to a hydrophilic polymer through a spacer comprising a structure derived from a group represented by -NH-R-L and/or a structure derived from an epoxy group. The spacer may be covalently bonded to the hydrophilic polymer and the ligand in the coating layer. This allows the ligand to be immobilized to the separation material. The spacer may be a low molecular or high molecular compound having, in a molecule, at least one each of functional groups that can react with a functional group of the hydrophilic polymer in the coating layer and a functional group of the ligand, respectively.

For example, in the case of using a ligand having an amino group such as protein A, examples of the method for immobilizing the ligand include reacting a hydrophilic polymer, which contains a functional group such as an epoxy group and a carboxy group to form a covalent bond with an amino group, directly with the ligand.

Further, examples of other immobilization methods include a method comprising reacting an amino group region of amino acids (amine carboxylic acids) for use as a spacer with an epoxy group of the hydrophilic polymer and then reacting a carboxy group on the other end with an amino group in the ligand, and a method comprising sequentially using a diamine or a diol and a diglycidyl compound such as (poly)ethylene glycol diglycidyl ether as spacer so as to bond one end of the diamine or the diol to the epoxy group of the separation material, another end to one of the epoxy groups of the diglycidyl compound, and the remaining epoxy group on the end to the ligand.

Examples of the diamines for use as a part of components of the spacer in the method include aliphatic diamines such as tetramethylenediamine and hexamethylene diamine. Examples of the diols include aliphatic diols such as propylene glycol, butanediol, diethylene glycol, triethylene glycol and polyethylene glycols.

It is preferable that the spacer have a straight chain structure in view of the reactivity with the ligand and the steric hindrance with the hydrophobic polymer particles in immobilization. With use of a spacer having a straight chain structure rather than a branched chain structure, the steric hindrance is reduced not to obstruct the formation of an affinity bond between the ligand and the antibody, so that the adsorption capacity tends to increase.

From the viewpoint of avoiding steric hindrance with an adjacent ligand, the spacer length is preferably 3 atoms or more and less than 50 atoms, more preferably 4 atoms or more and less than 50 atoms, still more preferably 4 atoms or more and less than 30 atoms, furthermore preferably 4 atoms or more and less than 20 atoms. The spacer length may be 4 atoms or more and less than 10 atoms. For example, when the hydrophilic polymer is a polysaccharide or a modified product thereof, the length of the spacer is represented by the number of atoms in the range from the oxygen atom derived from the hydroxy group in the hydrophilic polymer to the atom bonded to the ligand.

It is preferable that the amount of the functional group for immobilizing the ligand (e.g., epoxy group, carboxy group and amino group) be 0.01 to 500 $\mu$mol per mL of the hydrophobic polymer particles. With a content of 0.01 $\mu$ equivalents or more, the amount of the ligand immobilized can be increased, so that the immobilization of the ligand can be enhanced. With a content of 500 $\mu$ equivalents or less, the mobility of the ligand is enhanced, so that the amount of an antibody adsorbed tends to increase.

The ligand for use in the present embodiment has an affinity adsorptivity. Examples of such ligands include protein A, protein G, protein L and functional variants thereof, various antibodies, lectins, or pseudopeptide ligands thereof. The ligand is not particularly limited so long as it is a material with a biochemical activity having affinity for proteins and being able to be immobilized.

Among the ligands, one or more selected from protein A, protein G, protein L and functional variants thereof are preferred due to having a high selectivity when used for separation of antibodies. When the separation of antibodies is a main purpose, a ligand specifically bondable to a portion of an immunoglobulin is preferred.

The immobilization reaction of ligands can be performed, for example, by supplying an aqueous solution containing the ligand onto the coated particles having the reactive functional group to cause a reaction. It is preferable that the immobilization reaction temperature be about 4 to 30°C. Controlling the immobilization reaction temperature to 30°C or less allows the activity of the ligand to be easily maintained.

It is preferable that the immobilization density of the ligand per L of the separation material be more than 1 g. The upper limit thereof is not particularly limited, usually 50 g/L or less. With an immobilization density of the ligand of 1 g/L or more, the amount of an antibody adsorbed increases, so that the efficiency of the separation material can be increased. With an immobilization density of 50 g/L or less, the utilization efficiency of the ligand can be increased.

The amount of an antibody adsorbed on the separation material according to the present embodiment may be, for example, 5 mg or more per mL of the separation material. The amount of an antibody adsorbed is more preferably 10 mg or more, still more preferably 20 mg or more, furthermore preferably 40 mg or more, per mL of the separation material. Examples of the method for achieving such a high adsorption capacity include adjusting the immobilization density of the ligand, adjusting the pore diameter, adjusting the average particle size, and selecting the type of ligand having affinity adsorptivity, and two or more of the methods may be combined on an as needed basis. The preferred ranges of physical properties thereof are as described above.

In order to manufacture the separation material produced in an aseptic state as a separation material for use in an aseptic process, it is preferable that the hydrophobic polymer particles coated with the hydrophilic polymer be washed and sterilized with 40 vol% or more and 98 vol% or less of aqueous ethanol solution prior to immobilizing the ligand with a covalent bond, and the step of immobilizing the ligand be performed in a container where the sterilized state is maintained.

As the chemicals for washing and sterilization, various organic solvents other than ethanol may be also used. Examples of the chemicals other than ethanol include methanol, 2-propanol, 1-propanol, 1-butanol, aqueous formalin solution, acetone, formic acid, and acetic acid. Further, a commonly used washing and sterilizing agent such as a hydrogen peroxide solution, a hypochlorite aqueous solution, a sodium bicarbonate water, a sodium carbonate aqueous solution, saline or an alkaline aqueous solution (sodium hydroxide, potassium hydroxide or calcium hydroxide) may be used. The chemicals described above may each be mixed at any ratio for use.

The washing may be performed at any temperature, preferably at 2°C to 50°C. With a washing temperature in the range, the separation material can be prevented from being crushed due to freezing and from being decomposed due to high temperature. Although the washing and sterilization step may be performed after immobilization of the ligand, it is preferable that the washing and sterilization step be performed before immobilization of the ligand in order to eliminate the influence of ethanol or chemicals on the ligand activity. It is preferable that post-processes be then performed in an environment where the sterilized state is maintained.

As the sterilization method performed prior to immobilizing the ligand to the hydrophobic polymer particles coated with the hydrophilic polymer through a covalent bond, a method of making a sterilized state in the reaction step prior to immobilization of the ligand, and then performing the ligand immobilization step while maintaining the sterilized state is preferably used. Examples of the reaction step prior to immobilization of the ligand include a step of introducing a spacer. In the case of using an alkali catalyst in the spacer introduction step, the alkali concentration in the reaction solution may reach 0.1 mol/L to 10 mol/L, so that the separation material is sterilized in the reaction solution. Examples of the alkali catalyst added on this occasion include hydroxides such as sodium hydroxide, potassium hydroxide and calcium hydroxide, sodium carbonate, or sodium bicarbonate.

The reaction time in the spacer introduction step is usually 0.1 to 100 hours, preferably 0.1 to 5 hours. The temperature at which no freezing occurs is preferred, being 4°C to 200°C, preferably 10°C to 50°C. In the case of using a hydroxide such as sodium hydroxide in the step, use in an aqueous solution form or an alcohol solution form is preferred, so that it is preferable to use a liquid dissolving the hydroxide. In the case of using the hydroxide in an aqueous solution form, the range of the alkali concentration is preferably 0.1 mol/L to 10 mol/L, more preferably 0.5 mol/L to 5 mol/L. Within the range, the hydrophobic polymer particles are prevented from being hydrolyzed, so that properties can be improved.

After the immobilization reaction is performed as described above, it is preferable that the reactive functional groups remaining on the hydrophilic polymer be inactivated by a post-treatment. Through inactivation of the remaining reactive functional groups, a reaction with an active group of the ligand such as protein A is prevented, so that the adsorption capacity of the separation material and the selectivity can be improved.

In the case of having an epoxy group as the reactive functional group, examples of the post-treatment include an inactivation method based on reaction with an aqueous solution of amines such as ethanolamine. In the inactivation, treatment conditions such as the concentration of ethanolamine and the pH are not particularly limited, and the treatment can be usually performed under conditions at a concentration of 0.1 to 5 mol/L and at pH 7 to 14. With conditions in the ranges, the reaction rate of ethanolamine can be controlled to a practical range and deactivation of ligands such as protein A can be suppressed, which are preferred. Further preferred treatment conditions are those at a concentration of 1 to 2 mol/L and at pH 8 to 9.

It is preferable that the separation material after immobilization reaction of ligands, or the separation material after further post-treatment, be washed with water to remove unreacted materials. In the washing, it is more preferable that the washing be performed by alternate use of acidic washing water and basic washing water. On this occasion, it is still more preferable that washing be performed with alternate use of two types of buffer solutions, i.e., a buffer solution at pH 0 to 5 and a buffer solution at pH 8 to 15, which allows the excess ligands to be removed and the immobilized ligands to be activated.

Examples of the buffer solution usable for washing include acidic buffer solutions and basic buffer solutions. Examples of the acidic buffer solutions include a solution containing hydrochloric acid/potassium chloride, tartaric acid, citric acid, glycine, formic acid, acetic acid, succinic acid, phosphoric acid, and a salt thereof. Examples of the basic buffer solutions include a solution containing triethanolamine, tris(hydroxymethyl)aminomethane, diethanolamine, boric acid, ammonia, carbonic acid, and a salt thereof.

The ionic strength of the buffer solution used is preferably 0.001 M to 10 M, more preferably 0.01 M to 2 M. Using a buffer solution having an ionic strength in the range is preferred, allowing the deactivation of immobilized ligands to be reduced. The buffer solution used may contain a salt such as sodium chloride and potassium chloride. The presence of the salt is preferred, allowing removal of excess ligands and activation of immobilized ligands to be effectively performed. The concentration of the salt may be, for example, 0.1 to 2 M, preferably 0.5 to 1 M.

When the ligand-containing aqueous solution is supplied onto the coated particles having the reactive functional group to cause a reaction, the immobilization density of the ligand can be measured through determination of the ligand concentration in the supernatant before and after the reaction by HPLC or absorptiometry.

The resulting separation material may be temporarily stored, except when directly used. It is preferable to use an ethanol aqueous solution having a concentration of 1 to 50 mass% as the medium for storage. With the concentration of ethanol being controlled in the range, deactivation of the immobilized ligands can be suppressed. Further, storing the separation material according to the present embodiment in the above medium is preferable, because the swelling degree of the separator material becomes suitable and the affinity of the separation material to the storage medium is good. Use of the above medium is preferable, having an effect of suppressing growth of bacteria and improving the storage stability of the ligands immobilized in pores. The more preferred concentration of ethanol is 10 to 30 mass%, and the still more preferred concentration is 15 to 25 mass%.

It is preferable that the separation treatment of a target molecule using the separation material described above be performed such that the following step (a) and step (b) are included. In other words, the method for separating a target molecule according to the present embodiment includes the following step (a) and step (b):

(a) a step of contacting a solution containing a target molecule with the separation material, so that the target molecule is adsorbed on the separation material, and
(b) a step of eluting the target molecule from the separation material on which the target molecule is adsorbed.

According to the method described above, it is possible to separate the various proteins described above with high selectivity.

Examples of the target molecules include at least a portion of an immunoglobulin or a modified product thereof (e.g., chemically modified product). It is preferable that the immunoglobulin be one or more selected from the group consisting of a monoclonal antibody and a polyclonal antibody. It is more preferable that the target molecule be a fusion protein containing at least a portion of the Fc region of the immunoglobulin or a modified product thereof (e.g., chemically modified product).

In the separation treatment described above, a column of liquid chromatography comprising the separation material as a packing material and at least one container is suitably used.

It is preferable that the separating material of the present embodiment comprise 30 to 500 mg of a coating layer per g of hydrophobic polymer particle. The amount of the coating layer can be measured based on the weight loss in thermal decomposition or the like. It is more preferable that the amount of the coating layer be 30 to 400 mg per g of hydrophobic polymer particle.

The liquid passing rate in the present specification represents the liquid passing rate when a liquid is passed through a $\varphi$ 7.8-mm $\times$ 300-mm stainless steel column packed with the separation material of the present embodiment. When water is passed through the separation material of the present embodiment packed in the column such that the column has a pressure of 0.3 MPa, the water passing rate is preferably 500 cm/h or more, more preferably 800 cm/h or more. When separation of proteins is performed by column chromatography, the liquid passing rate of a protein solution or the like is usually in the range of 400 cm/h or less. In contrast, in the case of using the separation material of the present embodiment, a liquid passing rate of 500 cm/h or more can be achieved, being faster than those of conventional separation materials for protein separation.

The elastic modulus at 5% compressive deformation of the separation material in water (elastic modulus for 5% compression deformation in a wet state) may be, for example, 70 MPa or more, 75 MPa or more, 80 MPa or more, 100 MPa or more, or 110 MPa or more. The upper limit of the elastic modulus at 5% compressive deformation is not particularly limited. With an elastic modulus for 5% compression deformation in the range, the flexibility of the hydrophobic polymer particles is reduced so that deformation of the particles can be suppressed, and when used in a column, compaction is prevented so that increase in column pressure can be prevented. As a result, even at a high flow rate, high adsorption capacity purification can be performed. Further, with an elastic modulus of the separation material in the range, liquid passing properties and durability can be achieved.

The elastic modulus at 5% compressive deformation of the separation material of the present embodiment in water can be calculated as follows.

Using a micro-compression tester (manufactured by Fisher), a separation material immersed beforehand in water is compressed to 50 mN with a smooth end face of a square pillar (50 $\mu$m by 50 $\mu$m) at a loading rate of 1 mN/s under room temperature (25°C) conditions to measure the load and the compressive displacement. Based on the measurement values obtained, the elastic modulus of the separation material when compressively deformed by 5% (5% K-value) can be determined from the following equation. Also, the load at which a maximum change in the displacement occurs in the measurement is regarded as fracture strength (mN).

$$5\% \text{ K-value (MPa)} = (3/2^{1/2}) \cdot F \cdot S^{-3/2} \cdot R^{-1/2}$$

F: load (mN) when separation material is compressively deformed by 5%
S: compression displacement (mm) when separation material is compressively deformed by 5%
R: radius of separation material (mm)

The average pore size, the specific surface area and the like of the separation material can be adjusted by appropriately selecting the raw materials of hydrophobic polymer particles, the porosity forming agent, the hydrophilic polymer having hydroxy groups and the like.

The average pore size, the mode pore size, the specific surface area and the porosity of the separation material or the hydrophobic polymer particles of the present embodiment are values measured as follows by a mercury intrusion porosimeter (Autopore, manufactured by Shimadzu Corporation). About 0.05 g of a sample is added in a standard 5-mL powder cell (stem volume: 0.4 mL), and the measurement is performed under conditions at an initial pressure of 21 kPa (about 3 psia, equivalent to a pore diameter of about 60 $\mu$m). As the mercury parameters, the default mercury contact angle of the device is set to 130 degrees, and the mercury surface tension is set to 485 dynes/cm. Also, the pore size was limited to a range of 0.1 to 3 $\mu$m to calculate the respective values.

Into the separation material of the present embodiment, for example, an ion exchange group may be introduced through the functional group or apart from the functional group.

The separation material of the present embodiment can be suitably used for ion exchange purification, affinity purification, separation of proteins by electrostatic interaction, and the like. For example, by adding the separation material of the present embodiment into a mixed solution containing proteins so as to adsorbing only the proteins on the separation material by electrostatic interaction, then filtering the separation material from the solution, and adding the separation material into an aqueous solution having a high salt concentration, the proteins adsorbed on the separation material can be easily desorbed and collected. Further, it is possible to use the separation material of the present embodiment in column chromatography such as liquid chromatography.

As biopolymers which can be separated by using the separation material of the present embodiment, water-soluble substances are preferred. Specific examples include proteins such as blood proteins including serum albumin and immunoglobulins, enzymes present in a living body, protein bioactive substances produced by biotechnology, DNA, and biopolymers such as bioactive peptides. The molecular weight of the biopolymer is preferably 2,000,000 or less, more preferably 500,000 or less. Also, it is necessary to select the properties of the separation material, the conditions, etc., depending on the isoelectric point and the ionized state, etc., of the protein by a known method. Examples of the known method include a method described in Patent Literature 7 and the like.

In the separation of biopolymers such as proteins, the separation material of the present embodiment has advantages of particles of natural polymer and particles of synthetic polymer, respectively. The hydrophobic polymer particles in the separation material of the present embodiment have durability and alkali resistance. Further, the separation material of the present embodiment reduces the non-specific adsorption of proteins, so that adsorption and desorption of proteins tend to easily occur. Furthermore, the separation material of the present embodiment, tends to have a large adsorption capacity of proteins or the like under the same flow rate (dynamic adsorption capacity).

**Examples**

The present invention is described based on Examples as follows, though the present invention is not limited to the following Examples.

<Test Example 1>

(Hydrophobic polymer particles) In a 500-mL three neck flask, 16 g of divinyl benzene with a purity of 96% (manufactured by Nippon Steel & Sumikin Chemical Co., Ltd., trade name "DVB960"), 16 g of hexanol, 16 g of diethylbenzene and 0.64 g of benzoyl peroxide were added to an aqueous solution of 0.5 mass% of polyvinyl alcohol, and after emulsification using a micro-process server, the resulting emulsion was transferred to a flask and stirred for about 8 hours using a stirrer while heated in a water bath at 80°C. The resulting particles were filtered, and then washed with acetone to obtain hydrophobic polymer particles.

(Modified agarose) To 480 mL of an aqueous solution of 2 mass% agarose, 0.98 g of sodium hydroxide and 4.90 g of glycidyl phenyl ether were added to cause a reaction at 60°C for 6 hours, so that a phenyl group was introduced to agarose. The resulting modified agarose was reprecipitated with isopropyl alcohol and washed. The content of hydrophobic groups in the modified agarose calculated by the following method was 14.2%.

A powder agarose in a dry state (unmodified agarose) and an agarose with hydrophobic groups introduced, dried to a volatile matter content of less than 0.1 mass%, were dissolved in pure water at 70°C to prepare 0.05 mass% of aqueous solutions, respectively. The concentrations were obtained by measuring the absorbance of the respective aqueous solutions at 269 nm with a spectrophotometer, so that the hydrophobic group content was obtained based on the following equation.

·

$$\text{Hydrophobic group content (\%)} = (C_{AG}/(C_{HAG} + C_{AG})) \times 100$$

·

$C_{AG}$: Concentration of modified agarose structural unit

$$(mmol/L) = A/\varepsilon_{GPE} \times 1000$$

- $C_{HAG}$:

Concentration of unmodified agarose structural unit

$(mmol/l) = $ (Concentration of unmodified agarose structural unit

$(g/L)/$Agarose structural unit $(306$ g/mol$)) \times 1000$

- A:

True absorbance of modified agarose $=$ Absorbance of modified agarose - Absorption of unmodified agarose

- $\varepsilon_{GPE}$:

Absorption coefficient of glycidyl phenyl ether $= 1372$

$(L/(mol \cdot cm))$

- Concentration of unmodified agarose structural unit $(g/L) =$

Sample concentration of unmodified agarose unit (mass%) $\times$ 10 -

Concentration of modified agarose structural unit $(g/l)$

-

Absorption of unmodified agarose unit $=$ Absorbance of

unmodified agarose $\times$ (Sample concentration of modified agarose

$(mmol/L)/$Sample concentration of unmodified agarose $(mmol/L))$

- Concentration of modified agarose structural unit $(g/L) = (C_{AG}$

$\times$ modified agarose structural unit $(456$ g/mol$))/1000$

The hydrophobic group content of the modified agarose adsorbed on particles can be calculated in the same manner, by treating 0.2 g of particles in 10 mL of 1 M sulfuric acid at 70°C for 5 hours and determining the treatment solution concentration through measurement of the absorbance of the treatment solution at 269 nm with a spectrophotometer.

[Example 1]

<Formation of coating layer> To an aqueous solution of 20 mg/mL of a modified agarose, hydrophobic polymer particles were added at a concentration of 70 mL/g of particles and stirred at 55°C for 24 hours. After the modified agarose was adsorbed on the hydrophobic polymer particles, the particles were filtered and washed with hot water.

(Crosslinking treatment) The modified agarose adsorbed on hydrophobic polymer particles was crosslinked as follows. In a 0.4 M sodium hydroxide aqueous solution, 10 g of particles with modified agarose adsorbed were dispersed, and 0.02 M epichlorohydrin was added thereto to be stirred at room temperature for 24 hours. Then, after washing with a hot aqueous solution of 2 mass% dodecyl sodium sulfate and subsequent washing with pure water, coated particles having a coating layer of crosslinked modified agarose were obtained.

(Evaluation on ability of non-specific adsorption of protein) To 20 mL of a tris-HCl buffer solution (pH 8.0) with a BSA (Bovine Serum Albumin) concentration of 24 mg/mL, 0.2 g of the resulting coated particles were added and stirred at room temperature for 24 hours. Then, the supernatant was collected by centrifugation to determine the BSA concentration of the supernatant with a spectrophotometer. Based on the BSA concentration of the supernatant, the amount of BSA adsorbed on the particles was calculated. The BSA concentration was determined based on the absorbance at 280 nm by a spectrophotometer. Particles with a non-specific adsorption capacity of 1 mg/mL or less were regarded to be "in absence of non-specific adsorption", and particles with a non-specific adsorption capacity of 5 mg/mL or more were regarded to be "in presence of non-specific adsorption." The results are shown in Table 1.

(Introduction of epoxy group) Into 10 mL of a 0.4 M sodium hydroxide aqueous solution, the coated particles (dry weight: 1 g) obtained by filtering an aqueous suspension containing the coated particles was added, and 0.6 g of epichlorohydrin was further added thereto to allow a reaction to proceed for 3 hours while stirring at room temperature. After completion of the reaction, particles having epoxy groups were obtained through filtration and washing with 500 mL of water.

(Introduction of carboxylic group) Into a dispersion comprising the particles having epoxy groups dispersed in a 0.1 M boric acid-KCl buffer solution with pH 10, 11.5 g of 6-amino hexanoic acid was added, and after adjusting to pH 13 with NaOH (50 wt% solution), a reaction proceeded at 50°C for 5 hours. After completion of the reaction, the excess 6-aminohexanoic acid was removed by washing with water, so that a separation material having carboxy groups was obtained. The particle size of the separation material was measured with a flow type particle size measuring device, and the average particle size was calculated. The results are shown in Table 2.

(Measurement of amount of carboxy group) The amount of functional groups of the resulting separation material was measured as follows. To 0.2 g of the separation material in a wet state, 20 g of a 0.1 mol/L sodium hydroxide aqueous solution was added and stirred at room temperature for 30 minutes. After stirring, the mixture was left to stand, and 10 g of the supernatant was sampled to obtain a liquid (1) diluted to 30 g with ultrapure water. Further, a dispersion containing the separation material in a wet state was diluted to 30 g with ultrapure water to obtain a liquid (2). Subsequently, to each of the liquid (1) and the liquid (2), a 0.1 mol/L hydrochloric acid aqueous solution was added dropwise to pH 2. The amount of the functional group introduced was calculated from the following equation. In the equation, f represents the factor of the hydrochloric acid aqueous solution. The results are shown in Table 1.

$$\text{Amount of functional group introduced (mmol/g of particles)} =$$
$$0.1 \times f \times (\text{Amount dropped in liquid (2)} - \text{Amount dropped in liquid}$$
$$(1))/\text{Dry mass of separation material} \times 1/2$$

(Elastic modulus at 5% compressive deformation) The elastic modulus at 5% compressive deformation of the separation material in water was measured by the method described above. The results are shown in Table 2.

(Evaluation on column properties) A slurry of the resulting separation material with a concentration of 30 mass% (solvent: methanol) was packed in a φ 7.8-mm × 300-mm stainless steel column over 15 minutes. Then, water was passed through the column while changing the flow rate to measure the relation between the flow rate and the column pressure, so that liquid passing rate (linear flow rate) at 0.3 MPa was measured. The results are shown in Table 2.

[Example 2] A separation material having carboxy groups was prepared in the same manner as in Example 1, except that 6-aminohexanoic acid was replaced with 7.8 g of 3-amino-propanoic acid (β-alanine), and evaluation was performed in the same manner as in Example 1.

[Example 3] A separation material having carboxy groups was prepared in the same manner as in Example 1, except that 6-aminohexanoic acid was replaced with 9.0 g of 4-amino-butanoic acid, and evaluation was performed in the same manner as in Example 1.

[Example 4] A separation material having carboxy groups was prepared in the same manner as in Example 1, except that 6-aminohexanoic acid was replaced with 10.3 g of 5-amino-pentanoic acid (valeric acid), and evaluation was performed in the same manner as in Example 1.

[Example 5] A separation material having carboxy groups was prepared in the same manner as in Example 1, except that 6-aminohexanoic acid was replaced with 12.7 g of 7-amino-heptanoic acid, and evaluation was performed in the same manner as in Example 1.

[Example 6] Using the particles having epoxy groups prepared in Example 1 as separation material, evaluation was performed in the same manner as in Example 1. The amount of epoxy groups introduced was measured as follows.

(Measurement of amount of epoxy group) To 0.5 g of particles in a wet state, 10 g of water and 1 g of a 1N hydrochloric acid aqueous solution were added and stirred at 70°C for 1 hour to allow a reaction to proceed. After the reaction, the amount of epoxy groups introduced (mmol/g of particle) was measured by neutralization titration with 0.1 N sodium hydroxide.

[Example 7] A separation material having amino groups was prepared in the same manner as in Example 1, except that 6-aminohexanoic acid was replaced with 5.3 g of (anhydrous) ethylene diamine. The measurement of the amount of the amino group introduced was performed by the same method as in the measurement of the amount of the carboxy group introduced.

[Example 8] A separation material having amino groups was prepared in the same manner as in Example 1, except that 6-aminohexanoic acid was replaced with 6.5 g of 1,3-diaminopropane.

[Example 9] A separation material having amino groups was prepared in the same manner as in Example 1, except that 6-aminohexanoic acid was replaced with 7.7 g of 1,4-diaminobutane.

[Example 10] A separation material having amino groups introduced was prepared in the same manner as in Example 1, except that 6-aminohexanoic acid was replaced with 10.2 g of 1,6-diaminohexane.

[Example 11] A separation material having amino groups was prepared in the same manner as in Example 1, except that 6-aminohexanoic acid was replaced with 11.4 g of 1,7-diaminoheptane.

[Example 12] A separation material having amino groups was prepared in the same manner as in Example 1, except that 6-aminohexanoic acid was replaced with 12.6 g of 1,8-diaminooctane.

[Comparative Example 1] Commercially available CaptoDEAE (manufactured by GE Healthcare) was used as separation material.

[Comparative Example 2] The hydrophobic polymer particles prepared in Example 1 were used as separation material.

(Immobilization of ligand) To 0.09 g of the separation material having carboxy groups in Examples 1 to 5, 0.02 g protein A, 0.02 g of WSC (1-ethyl-3-(3-dimethylaminopropyl) carbodiimide hydrochloride) and 2 mL of 50 mM PBS adjusted to pH 5.8 were added, and after stirring at room temperature for 24 hours, the ligand-immobilized particles were produced by filtration and washing, respectively.

To 0.09 g of the separation material having epoxy groups in Example 6, 0.02 g of protein A and 2 mL of a 0.5 M carbonate buffer solution (prepared from sodium hydrogen carbonate and sodium carbonate manufactured by Wako Pure Chemical Industries, Ltd., and water) at pH 10 were added, and after stirring at 30°C for 24 hours, the ligand-immobilized particles were produced by filtration and washing.

(Calculation of amount of ligand immobilized) The particles were filtered from the reaction solution after stirring for 24 hours, a sample containing ligand-immobilized particles having a predetermined concentration was then prepared. The absorbance (280 nm) of the sample was measured, and the amount of the ligand immobilized was calculated based on a calibration curve prepared in advance. The results are shown in Table 2.

[Table 1]

|  | Hydrophobic polymer particle | Hydrophilic polymer | Non-specific adsorption | Functional group | Amount of functional group introduced (mmol/g particle) |
|---|---|---|---|---|---|
| Example 1 | Present | Present | Absent | Carboxy group | 0.13 |
| Example 2 | Present | Present | Absent | Carboxy group | 0.14 |
| Example 3 | Present | Present | Absent | Carboxy group | 0.14 |
| Example 4 | Present | Present | Absent | Carboxy group | 0.13 |

(continued)

| | Hydrophobic polymer particle | Hydrophilic polymer | Non-specific adsorption | Functional group | Amount of functional group introduced (mmol/g particle) |
|---|---|---|---|---|---|
| Example 5 | Present | Present | Absent | Carboxy group | 0.13 |
| Example 6 | Present | Present | Absent | Epoxy group | 0.20 |
| Example 7 | Present | Present | Absent | Amino group | 0.14 |
| Example 8 | Present | Present | Absent | Amino group | 0.14 |
| Example 9 | Present | Present | Absent | Amino group | 0.13 |
| Example 10 | Present | Present | Absent | Amino group | 0.13 |
| Example 11 | Present | Present | Absent | Amino group | 0.13 |
| Example 12 | Present | Present | Absent | Amino group | 0.13 |
| Comparative Example 1 | Absent | Present | Absent | Amino group | 1.3 |
| Comparative Example 2 | Present | Absent | Present | - | - |

[Table 2]

| | Elastic modulus (MPa) | Liquid passing rate (cm/h) | Average particle size ($\mu$m) | Amount of Protein A immobilized (g/g particle) |
|---|---|---|---|---|
| Example 1 | 90 | 1290 | 119 | 0.23 |
| Example 2 | 100 | 1280 | 120 | 0.25 |
| Example 3 | 90 | 1280 | 121 | 0.25 |
| Example 4 | 110 | 1290 | 119 | 0.23 |
| Example 5 | 90 | 1290 | 119 | 0.23 |
| Example 6 | 90 | 1290 | 119 | 0.35 |
| Example 7 | 110 | 1290 | 119 | - |
| Example 8 | 110 | 1280 | 119 | - |
| Example 9 | 110 | 1300 | 119 | - |
| Example 10 | 110 | 1280 | 119 | - |
| Example 11 | 90 | 1290 | 119 | - |
| Example 12 | 90 | 1300 | 119 | - |
| Comparative Example 1 | 59 | 250 | 90 | - |
| Comparative Example 2 | 140 | 1200 | 100 | - |

The separation material of in each of Examples was able to suppress non-specific adsorption, having a high elastic modulus at 5% compressive deformation and a high liquid passing rate at 0.3 MPa, being excellent in liquid passing properties when packed in a column. Further, the separation material in each of Examples 1 to 6 was able to immobilize protein A ligands due to introduction of functional groups. The separating material in each of Examples 7 to 12 can be used as a carrier bonding with ligands containing carboxy groups.

<Test Example 2>

(Example 13)

(Synthesis of hydrophobic polymer particles) In a 500-mL three neck flask, 16 g of divinyl benzene with a purity of 96% (manufactured by Nippon Steel & Sumikin Chemical Co., Ltd., trade name "DVB960") as monomer, 16 g of hexanol and 16 g of diethylbenzene as porosity forming agent, and 0.64 g of benzoyl peroxide as initiator were added to an aqueous solution of 0.5 mass% of polyvinyl alcohol dispersant, and after emulsification using a micro-process server, the resulting emulsion was transferred to a flask and stirred for about 8 hours using a stirrer while heated in a water bath at 80°C. The resulting particles were filtered, and then washed with acetone to obtain hydrophobic polymer particles. The particle size of the resulting hydrophobic polymer particles was measured with a flow type particle size measuring device, and the C. V value (coefficient of variation) of the particle size was calculated. The results are shown in Table 3.

(Introduction of hydrophobic group to hydrophilic polymer having hydroxy group) To 480 mL of an agarose aqueous solution (2 mass%), 0.98 g of sodium hydroxide and 4.90 g of glycidyl phenyl ether were added to allow a reaction to proceed at 60°C for 6 hours, so that phenyl groups were introduced to agarose. The resulting modified agarose was precipitated with isopropyl alcohol and washed. The hydrophobic group content of the modified agarose calculated in the same manner as in Test Example 1 was 14.2%.

(Modified polymer coating to hydrophobic polymer particles)

To an aqueous solution of 20 mg/mL of modified agarose, hydrophobic polymer particles are added at a concentration of 70 mL/g of particles and stirred at 55°C for 24 hours, so that the modified agarose was adsorbed on the hydrophobic polymeric particles. After adsorption, filtration and washing with hot water were performed.

(Crosslinking of modified polymer) The modified agarose adsorbed on the particle surface was crosslinked as follows. In a 0.4 M sodium hydroxide aqueous solution, 10 g of particles with modified agarose adsorbed were dispersed, and 0.04 M epichlorohydrin was added thereto to be stirred at room temperature for 8 hours. After that, the particles were washed with a hot aqueous solution of 2 mass% of dodecyl sodium sulfate, and then with pure water. After drying the resulting particles, the amount of coating of hydrophilic polymer was measured by thermogravimetric analysis as follows. The results are shown in Table 3.

The amount of coating (mg) per g of the hydrophobic polymer particles was calculated from TG at the temperature corresponding to 5% weight loss measured with a differential thermogravimetric analyzer (TG-DTA). The measurement was performed in the temperature range of 40 to 500°C after keeping temperature at 40°C for 30 minutes, with a temperature rising rate of 10°C/min. The amount of coating was calculated from the following equation.

$$\text{Amount of coating (mg/g of particle)} = R/(100 - R) \times 1000$$

$$R\ (\%) = 95\% - T$$

R (%): Ratio of amount of coating to separation material
T (%): TG of separation material at temperature corresponding to 5% weight loss of hydrophobic polymer particle

(Evaluation on non-specific adsorption of protein) To 20 mL of a tris-HCl buffer solution (pH 8.0) with a BSA (Bovine Serum Albumin, Mw 66,000) concentration of 24 mg/mL, 0.2 g of the resulting coated particles were added and stirred at room temperature for 24 hours. Then, the supernatant was collected by centrifugation to determine the BSA concentration of the supernatant with a spectrophotometer. Based on the BSA concentration of the supernatant, the amount of BSA adsorbed on the coated particles was calculated. The BSA concentration was determined based on the absorbance at 280 nm by a spectrophotometer. Particles with a non-specific adsorption capacity of 5 mg/mL or less were rated as A, and particles with a non-specific adsorption capacity of more than 5 mg/mL were rated as B. The results are shown in Table 5.

(Introduction of epoxy group) Into 10 mL of a 0.4 M sodium hydroxide aqueous solution, the coated particles (dry weight: 1 g) obtained by filtering an aqueous suspension containing the coated particles was added, and 0.6 g of epichlorohydrin was further added thereto to allow a reaction to proceed for 3 hours while stirring at room temperature. After completion of the reaction, the particles were filtered and washed with 500 mL of water to obtain a separation material having epoxy groups.

(Introduction of carboxy group) Into a dispersion comprising the separation materials having epoxy groups dispersed in a 0.1 M boric acid-KCl buffer solution with pH 10, 11.5 g of 6-amino hexanoic acid was added, and the mixture was adjusted to pH 13 with NaOH (50 wt% solution). A reaction then proceeded at 50°C for 5 hours. After the reaction, the excess 6-aminohexanoic acid was removed by washing with water, so that a separation material having carboxy groups was obtained.

(Immobilization of ligand) To 1 ml of the separation material having carboxy groups, 0.15 ml of an aqueous solution dissolving protein A with a concentration of 50 mg/ml, 0.02 g of WSC (1-ethyl-3-(3-dimethylaminopropyl)carbodiimide hydrochloride) and 5 mL of a 50 mM PBS buffer solution adjusted to pH 5.8 were added, and stirred at room temperature for 24 hours. The particles were then filtered and washed with water to remove unreacted protein A and WSC. The absorbance of the supernatant after stirring was measured to calculate the amount of the ligand immobilized based on a calibration curve prepared in advance. The separation material was dispersed in 10 ml of a 1 M ethanolamine aqueous solution, and after 0.1 g of WSC was added thereto, the mixture was stirred at room temperature for 4 hours. After that, the particles were filtered and washed with water to remove unreacted ethanolamine and WSC, so that a ligand-immobilized separation material with unreacted functional group blocked was obtained. The mode diameter and the specific surface area in the pore size distribution of the resulting particles were measured by mercury intrusion porosimetry. The results are shown in Table 3.

(Elastic modulus at 5% compressive deformation) The elastic modulus at 5% compressive deformation of the separation material in water was measured by the same method in Test Example 1. The results are shown in Table 5.

(Evaluation on column properties) A slurry of the resulting separation material with a concentration of 30 mass% (solvent: methanol) was packed in a $\varphi$ 7.8-mm $\times$ 300-mm stainless steel column over 15 minutes. Then, water was passed through the column while changing the flow rate to measure the relation between the flow rate and the column pressure, so that the column flow rate (liquid passing rate) at 0.3 MPa was measured. The results are shown in Table 5.

(Dynamic adsorption capacity) The dynamic adsorption capacity was measured as follows. Through a column, 10 column volumes of a 20 mmol/L PBS buffer solution (pH 7.2) was run. Thereafter, a 20 mmol/L PBS buffer solution having an IgG (immunoglobulin G) concentration of 5 mg/mL (pH 7.2) was run to measure the IgG concentration at the column outlet by UV measurement. The liquid was run until the IgG concentration at the column inlet matched with the IgG concentration at the column outlet, and after the unadsorbed fraction of IgG was eluted with 5 column volumes of a 20 mmol/L PBS buffer solution (pH 7.2), 10 column volumes of a 100 mmol/L citric acid buffer solution (pH 3.0) was run, so that the IgG adsorbed was collected. The dynamic binding capacity at 10% breakthrough (10% Dynamic Binding Capacity, hereinafter, referred to as 10% DBC) was calculated using the following equation. The flow rate was set such that the residence (residence time in column) was equal to 1 minute. The results are shown in Table 5.

$$q_{10} = c_f F(t_{10} - t_0)/V_B$$

$q_{10}$: dynamic binding capacity at 10% breakthrough (mg/mL wet resin)
$c_f$: injection concentration of IgG
F: flow rate (mL/min)
$V_B$: bed volume (mL)
$t_{10}$: time at 10% breakthrough (minute)
$t_0$: BSA injection start time (minute)

(Evaluation on alkali resistance) The resulting separation material was stirred in a 0.5 M sodium hydroxide solution for 24 hours, and after washing with a PBS buffer solution (pH 7.2), packed in a column under the conditions described above. The 10% breakthrough dynamic binding capacity of IgG was measured to compare it with the dynamic binding capacity before the alkali treatment. The case of a reduction in dynamic binding capacity of 3% or less was given a rating A; the case of a reduction in dynamic binding capacity of more than 3% and less than 11% was given a rating B; and the case of a reduction in dynamic binding capacity of 11% or more was given a rating C. The results are shown in Table 5.

(Evaluation on durability) Water was passed through a column at a flow rate of 800 cm/h, and after measurement of the column pressure, the liquid passing was performed for 1 hour at an increased flow rate of 3000 cm/h. When lowering the column pressure to 800 cm/h again, the case where the column pressure increased by more than 10% than the initial value (before increasing the flow rate to 3000 cm/h) was given a rating B; and the case the column pressure increased within 10% was given a rating A. The results are shown in Table 5.

(Example 14) A separation material was prepared in the same manner as in Example 13, except that 6-aminohexanoic acid was replaced with 9.0 g of 4-aminobutylic acid, and evaluation was performed.

(Example 15) A separation material was prepared in the same manner as in Example 13, except that 6-aminohexanoic acid was replaced with 7.8 g of 3-aminopropanoic acid, and evaluation was performed.

(Comparative Example 3) The ligands were immobilized by the method described above, using commercially available carboxy group-introduced particles (Sepharose ECH, manufactured by GE Healthcare) as agarose particles, so that evaluation of Comparative Example 3 was performed.

(Comparative Example 4) The ligands were immobilized by the method described above, using TOYOPEARL AF-Carboxy-650 manufactured by Tosoh Corporation which is resin particles of an acrylic polymer matrix, so that evaluation of Comparative Example 4 was performed.

(Comparative Example 5) A commercially available affinity purification carrier rProtein A Sepharose Fast Flow (manufactured by GE Healthcare Inc.) was directly used, so that evaluation of Comparative Example 5 was performed.

[Table 3]

| | Coating polymer | Amount of coating (mg/g particle) | Mode pore size ($\mu$m) | C. V. of particle size (%) | Specific surface area (m$^2$/g) |
|---|---|---|---|---|---|
| Example 13 | Agarose | 350 | 0.11 | 7.9 | 2.7 |
| Example 14 | | | | | |
| Example 15 | | | | | |
| Comparative Example 3 | - | - | - | 17 | - |
| Comparative Example 4 | - | - | - | 10 | - |
| Comparative Example 5 | | - | - | 15 | - |

[Table 4]

| | Amino acid in spacer | Number of atoms of spacer |
|---|---|---|
| Example 13 | 6-Aminohexanoic acid | 10 |
| Example 14 | 4-Aminobutylic acid | 8 |
| Example 15 | 3-Aminopropanoic acid | 7 |
| Comparative Example 3 | 6-Aminohexanoic acid | 10 |
| Comparative Example 4 | - | - |
| Comparative Example 5 | - | - |

[Table 5]

| | Amount of protein A immobilized (mg/ml) | Column flow rate at 0.3 Mpa (cm/h) | Elastic modulus (Mpa) | BSA non-specifi c adsorption (mg/mL) | 10% DBC (residence: 1 min) (mg/mL) | Alkali resistance | Durability |
|---|---|---|---|---|---|---|---|
| Example 13 | 6.5 | 2700 | 330 | 5 or less, A | 37 | A | A |
| Example 14 | 6.6 | 2700 | 270 | 5 or less, A | 36 | A | A |
| Example 15 | 6.4 | 2800 | 220 | 5 or less, A | 33 | A | A |

(continued)

|  | Amount of protein A immobilized (mg/ml) | Column flow rate at 0.3 Mpa (cm/h) | Elastic modulus (Mpa) | BSA non-specifi c adsorption (mg/mL) | 10% DBC (residence: 1 min) (mg/mL) | Alkali resistance | Durability |
|---|---|---|---|---|---|---|---|
| Comparative Example 3 | 6.2 | 650 | 60 | - | 22 | B | B |
| Comparative Example 4 | 6.3 | 1200 | 70 | - | 26 | B | B |
| Comparative Example 5 | 6 | 500 | 60 | - | 20 | B | B |

The separation material in each of Examples was able to suppress non-specific adsorption, having a high elastic modulus at 5% compressive deformation and a high liquid passing rate at 0.3 MPa.

**Claims**

1. A separation material comprising:

   hydrophobic polymer particles; and
   a coating layer covering at least a portion of a surface of the hydrophobic polymer particles, wherein
   the coating layer comprises a hydrophilic polymer having hydroxy groups, and the hydrophilic polymer has a group represented by -NH-R-L or an epoxy group, wherein R represents a hydrocarbon group and L represents a carboxy group or an amino group.

2. The separation material according to claim 1, wherein the separation material has an elastic modulus at 5% compressive deformation in water of 70 MPa or more.

3. The separation material according to claim 1 or 2, wherein when water is passed through a column packed with the separation material such that the column has a pressure of 0.3 MPa, a water passing rate is 500 cm/h or more.

4. The separation material according to any one of claims 1 to 3, wherein the hydrophobic polymer particles are particles comprising a polymer having a structural unit derived from a styrene monomer.

5. The separation material according to any one of claims 1 to 4, wherein the hydrophilic polymer having hydroxy groups is a polysaccharide or a modified product thereof.

6. The separation material according to any one of claims 1 to 5, wherein the hydrophilic polymer having hydroxy groups is one or more selected from the group consisting of dextran, agarose, pullulan, a modified product thereof, and a mixture thereof.

7. The separation material according to any one of claims 1 to 6, wherein the hydrophilic polymer having hydroxy groups is crosslinked.

8. The separation material according to any one of claims 1 to 7, wherein the separation material has an average particle size of 10 to 500 $\mu$m.

9. The separation material according to any one of claims 1 to 8, wherein the separation material has a mode diameter in pore size distribution of 0.05 to 0.6 $\mu$m.

10. The separation material according to any one of claims 1 to 9, wherein the separation material has a pore volume of 30 to 70 vol%.

11. The separation material according to any one of claims 1 to 10, wherein the hydrophobic polymer particles have a porous structure.

12. The separation material according to any one of claims 1 to 11, wherein the separation material has a specific surface area of 20 $m^2$/g or more.

13. The separation material according to any one of claims 1 to 12, wherein the hydrophobic polymer particles have a coefficient of variation of a particle size of 3 to 15%.

14. The separation material according to any one of claims 1 to 13, wherein an amount of the coating layer is 30 to 500 mg per g of the hydrophobic polymer particles.

15. The separation material according to any one of claims 1 to 14, wherein the coating layer further has a ligand bonded to the hydrophilic polymer through a spacer comprising a structure derived from the group represented by -NH-R-L or a structure derived from the epoxy group.

16. The separation material according to claim 15, wherein the ligand is covalently bonded to the spacer.

17. The separation material according to claim 15 or 16, wherein the spacer is in a straight chain form.

18. The separation material according to any one of claims 15 to 17, wherein the hydrophilic polymer is a polysaccharide or a modified product thereof, and a length of the spacer represented by the number of atoms from an oxygen atom derived from the hydroxy group in the hydrophilic polymer to an atom bonded to the ligand is 4 atoms or more and less than 50 atoms.

19. The separation material according to any one of claims 15 to 18, wherein the ligand is bondable specifically to a portion of an immunoglobulin.

20. The separation material according to any one of claims 15 to 19, wherein the ligand is one or more selected from the group consisting of protein A, protein G, protein L and functional variants thereof.

21. A column comprising the separation material according to any one of claims 1 to 20.

22. A method for separating a target molecule comprising:

(a) contacting a solution comprising a target molecule with the separation material according to any one of claims 1 to 20 to adsorb the target molecule on the separation material; and
(b) eluting the target molecule from the separation material on which the target molecule is adsorbed.

23. The method according to claim 22, wherein the target molecule is at least a portion or a modified product of an immunoglobulin.

24. The method according to claim 23, wherein the immunoglobulin is one or more selected from the group consisting of a monoclonal antibody and a polyclonal antibody.

25. The method according to any one of claims 22 to 24, wherein the target molecule is a fusion protein comprising at least a portion of an Fc region of an immunoglobulin, or a modified product thereof.

## INTERNATIONAL SEARCH REPORT

| | International application No. |
|---|---|
| | PCT/JP2018/013304 |

**A. CLASSIFICATION OF SUBJECT MATTER**

Int.Cl. B01J20/285(2006.01)i, B01D15/36(2006.01)i, B01D15/38(2006.01)i, B01J20/24(2006.01)i, B01J20/281(2006.01)i

According to International Patent Classification (IPC) or to both national classification and IPC

**B. FIELDS SEARCHED**

Minimum documentation searched (classification system followed by classification symbols)

Int.Cl. B01J20/285, B01D15/36, B01D15/38, B01J20/24, B01J20/281

| Documentation searched other than minimum documentation to the extent that such documents are included in the fields searched | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2016/117567 A1 (HITACHI CHEMICAL CO., LTD.) 28 July 2016, entire text, claims 1–8, paragraphs [0010], [0039]–[0082] & EP 3248677 A1, claims 1–8 | 1–15, 20–23<br>16–19, 24–25 |
| Y | JP 2016–534984 A (GLAXOSMITHKLINE INTELLECTUAL PROPERTY DEVELOPMENT LIMITED) 10 November 2016, fig. 1, paragraphs [0051]–[0055] & US 2016/0221962 A1, fig. 1, paragraphs [0132]–[0136] & WO 2015/049651 A1, fig. 1 & EP 3052483 A1, fig. 1 & CA 2925862 A1, fig. 1 | 16–19, 24–25 |

☒ Further documents are listed in the continuation of Box C.    ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 15 June 2018 (15.06.2018) | 26 June 2018 (26.06.2018) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2018/013304

C (Continuation). DOCUMENTS CONSIDERED TO BE RELEVANT

| Category* | Citation of document, with indication, where appropriate, of the relevant passages | Relevant to claim No. |
|---|---|---|
| A | JP 2015-509486 A (GRAFFINITY PHARMACEUTICALS GMBH) 30 March 2015, entire text & US 2016/0009760 A1 & WO 2013/117707 A1 & EP 2812346 A1 | 1-25 |
| A | JP 2010-534336 A (GE HEALTHCARE BIO-SCIENCES AB) 04 November 2010, entire text & US 2010/0320149 A1 & WO 2009/014481 A1 & EP 2167229 A1 & CA 2693334 A1 | 1-25 |
| A | WO 2012/020080 A2 (GRAFFINITY PHARMACEUTICALS GMBH) 16 February 2012, entire text (Family: none) | 1-25 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4965289 A
- US 3966489 A
- JP H01254247 A
- US 5114577 A
- JP 2009031277 A
- US 4308254 A
- JP S60169427 A